(19) Europäisches Patentamt
European Patent Office
Office européen des brevets

(11) Veröffentlichungsnummer: **0 277 910**
**A1**

# (12) EUROPÄISCHE PATENTANMELDUNG

(21) Anmeldenummer: **88730023.4**

(22) Anmeldetag: **29.01.88**

(51) Int. Cl.[4]: **C 07 J 1/00**
A 61 K 31/565

(30) Priorität: **04.02.87 DE 3703722**

(43) Veröffentlichungstag der Anmeldung:
**10.08.88 Patentblatt 88/32**

(84) Benannte Vertragsstaaten:
**AT BE CH DE ES FR GB GR IT LI LU NL SE**

(71) Anmelder: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Kerb, Ulrich, Dr.**
**Prinzregentenstrasse 7**
**D-1000 Berlin 31 (DE)**

**Nishino, Yukishige, Dr.**
**Lanzendorfer Weg 14**
**D-1000 Berlin 22 (DE)**

**Henderson, David, Dr.**
**Jahnstrasse 17**
**D-1000 Berlin 28 (DE)**

(54) **1-Methyl-15Alpha-(1,2-dialkanoyloxyalkyl)-androsta-1,4-dien-3,17-dione, Verfahren zu deren Herstellung und diese enthaltende pharmazeutische Präparate.**

(57) 1-Methyl-15α-alkyl-(1,2-dialkanoyloxy)-androsta-1,4-dien-3,17-dione der allgemeinen Formel I

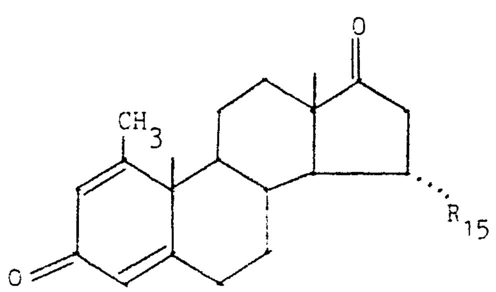

(I),

worin
$R_{15}$ eine geradkettige oder verzweigte Alkylgruppe mit 2 bis 10 Kohlenstoffatomen darstellt, die in 1- und 2-Stellung durch zwei $C_1$-$C_7$-Alkanoyloxygruppen, die gleich oder verschieden sein können, substituiert ist, sind als Inhibitoren der Östrogenbiosynthese zur Fertilitätskontrolle und zur Behandlung von Krankheiten geeignet, die durch Östrogene hervorgerufen werden.

EP 0 277 910 A1

**Beschreibung**

1-Methyl-15α-(1,2-dialkanoyloxyalkyl)-androsta-1,4-dien-3,17-dione, Verfahren zu deren Herstellung und diese enthaltende pharmazeutische Präparate

Die Erfindung betrifft 1-Methyl-15α-(1,2-dialkanoyloxyalkyl)-androsta1,4-dien-3,17-dione, Verfahren zu deren Herstellung, diese enthaltende pharmazeutische Präparate und ihre Verwendung zur Herstellung von Arzneimitteln.

In der deutschen Offenlegungsschrift 35 39 244 werden 1-Methyl-15α-alkyl-androsta-1,4-dien-3,17-dione der allgemeinen Formel

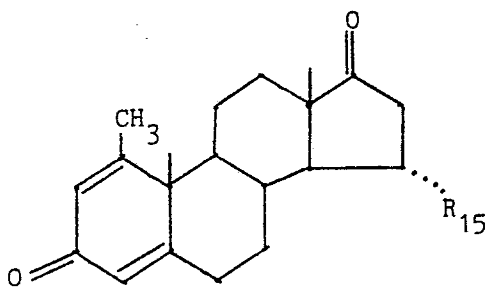

beansprucht,
worin,
$R_{15}$ eine Alkylgruppe mit 1 bis 4 Kohlenstoffatomen darstellt, die gegebenenfalls in 1- und/oder 2-Stellung durch eine Hydroxy- oder $C_2$-$C_7$-Alkanoyloxygruppe oder eine 1,2-Isopropylidendioxygruppe substituiert ist.

Die Verbindungen sind Inhibitoren der Östrogenbiosynthese (Aromatasehemmer). Sie sind damit zur Behandlung von Krankheiten geeignet, die durch Östrogene bdedingt oder von Östrogenen abhängig sind. So sind geeignet zur Behandlung von östrogeninduzierten oder -stimulierten Tumoren, wie zum Beispiel Mammakarzinom oder Prostatahyperplasie.

Die erfindungsgemäßen Verbindungen sind auch wertvoll zur Beeinflussung der Fertilität. So kann eine männliche Infertilität, die aus erhöhten Östrogenspiegeln resultiert, mit den neuen Wirkstoffen behoben werden.

Ferner können die Verbindungen bei Frauen im fortpflanzungsfähigen Alter als Antifertilitätsmittel verwendet werden, um Ovulation oder Implantation durch Östrogenentzug zu hemmen.

Wahrscheinlich sind Aromatasehemmer auch zur Behandlung des drohenden Herzinfarkts geeignet, da erhöhte Östrogenspiegel beim Mann einem Herzinfarkt vorangehen können.

Es hat sich nun überraschenderweise herausgestellt, daß die in 1,2-Position des 15α-Alkylsubstituenten veresterten Dihydroxy-Derivate der allgemeinen Formel I

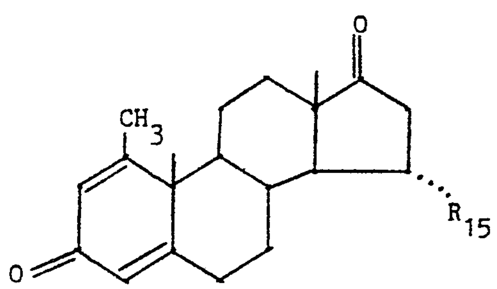

(I),

worin
$R_{15}$ eine geradkettige oder verzweigte Alkylgruppe mit 2 bis 10 Kohlenstoffatomen darstellt, die in 1- und 2-Stellung durch zwei gleiche oder verschiedene $C_1$-$C_7$-Alkanoyloxygruppen substituiert ist,
als Aromatasehemmer derartig herausragende Eigenschaften besitzen, daß damit die Dosierung gegenüber den bekannten Verbindungen um ein Mehrfaches herabgesetzt werden kann. Natürlich können dadurch auch unerwünschte Nebenwirkungen noch stärker zurückgedrängt werden.

Bevorzugte in 1 - und 2-Stellung alkanoyloxylierte Alkylgruppen der allgemeinen Formel I sind beispielsweise die Propyl-, Butyl-, 3-Methylbutyl-, Pentyl-, Heptyl- und Decylgruppen. Als $C_1$-$C_7$-Alkanoyloxygruppen finden vorzugsweise die Formyloxy-, Acetoxy-, Propinoyloxy- und Butyryloxygruppen Anwendung.

Im Vergleich zu dem bekannten Aromatasehemmer 4-Hydroxy-4-androsten-3,17-dion (A) und dem in der deutschen Offenlegungsschrift 35 39 244 beschriebenen Aromatasehemmer 15α-(Acetoxyethyl)-1-methyl-androsta-1,4-dien-3,17-dion (B) wird die Serum-östradiolkonzentration mit den erfindungsgemäßen Verbindungen, wie zum Beispiel 15α-(1R,2S-Diacetoxypropyl)-1-methyl-androsta-1,4-dien-3,17-dion (C) und

15α-(1R,2S-Diacetoxybutyl)-1-methyl-androsta-1,4-dien-3,17-dion (D), stärker gesenkt.

Infantile weibliche Ratten reagieren auf PMSG (Pregnant Mare's Serum Gonadotropin)-Behandlung mit einheitlich erhöhter Steroidsynthese. Die Aromataseaktivität kann durch die Beeinflussung der PMSG-stimulierten Östrogenbildung gemessen werden.

Im sogenannten PMSG-Test werden 21 tage alte weibliche Ratten alle 2 Tage insgesamt 7 mal mit je 100 I.U. PMSG subkutan vorbehandelt. 1 Stunde vor und 8 Stunden nach der letzten PMSG-Applikation($d_{12}$) erhalten die Tiere die Testsubstanz in 0,1 ml Benzylbenzoat/Rizinusöl (1+9) durch subkutane Injektion. Die Kontrolltiere erhalten nur das Vehikel. 24 Stunden nach der letzten PMSG-Applikation werden die Tiere getötet. Im serum wird Östradiol radioimmunologisch geprüft. Für jede Gruppe von Tieren wird der Mittelwert der Östradiolkonzentration in nMol/l mit der Standardabweichung errechnet. Die Signifikanzen der Unterschiede zur Kontrollgruppe werden durch die Varianzanalyse geprüft.

Zur Ermittlung der relativen Wirkungsstärke der zu testenden Substanz im Vergleich zu der Standardsubstanz wird die Regressions- und Kovarianzanalyse durchgeführt. Ferner wird die prozentuale Hemmung gegen die PMSG-Kontrolle berechnet.

Tabelle

**Beeinflussung der Östradiol-Konzentration im peripheren Serum bei der PMSG-vorbehandelten Ratte**

| | Dosis mg/Tier 2 x s.c. | n | Östradiol-Konzentration nMol/l | Hemmung % |
|---|---|---|---|---|
| PMSG-Kontrolle | - | 10 | 3,42±0,56 | - |
| | 0,1 | 10 | 3,98±1,12 | - |
| A | 0,3 | 10 | 3,84±0,84 | - |
| | 1,0 | 10 | 1,94±0,62 | 44 |
| | 0,1 | 10 | 2,11±0,39 | 38 |
| B | 0,3 | 10 | 0,62±0,13 | 82 |
| | 1,0 | 10 | 0,54±0,07 | 84 |
| | 0,1 | 10 | 1,30±0,37 | 62 |
| C | 0,3 | 10 | 0,68±0,32 | 80 |
| | 1,0 | 10 | 0,24±0,10 | 93 |
| | 0,1 | 10 | 0,54±0,11 | 84 |
| D | 0,3 | 10 | 0,88±0,15 | 74 |
| | 1,0 | 10 | 0,41±0,09 | 88 |

n = Tierzahl pro Gruppe

Gegenüber den in der deutschen Offenlegungsschrift 35 39 244 beschriebenen Verbindungen zeigen die erfindungsgemäßen Verbindungen bei subkutaner Applikation eine 3-10 mal stärkere Wirksamkeit.

Die Verbindungen der Formel I werden in der deutschen Offenlegungsschrift 35 39 244 nicht beschrieben.

Die Erfindung betrifft auch Verfahren zur Herstellung von Verbindungen der allgemeinen Formel I, dadurch gekennzeichnet, daß man

a) die nach der deutschen Offenlegungsschrift 35 39 244 darstellbaren 1-Methyl-15α-alkyl-androsta-1,4-dien-3,17-dione der allgemeinen Formel II

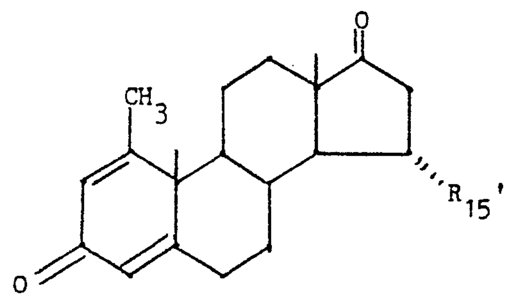

(II),

worin

$R_{15}'$ eine Alkylgruppe mit 2 bis 10 Kohlenstoffatomen darstellt, die in 1- und 2-Stellung durch zwei Hydroxygruppen substituiert ist,

verestert oder

b) die nach der deutschen Offenlegungsschrift 35 39 244 herstellbaren 15α-(1,2-Epoxyalkyl)-1α-methyl- 5α-androstan-3-one der allgemeinen Formel III

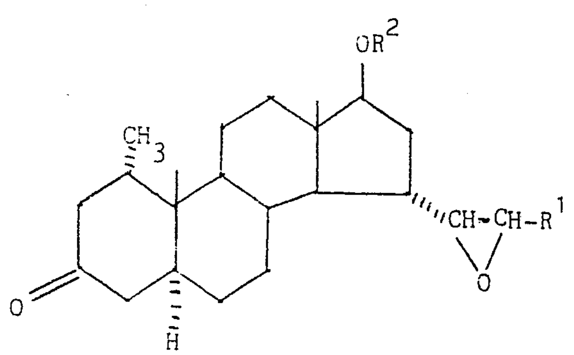

(III) ,

worin

$R^1$ für ein Wasserstoffatom oder eine geradkettige oder verzweigte Alkylgruppe mit bis zu 8 Kohlenstoffatomen und $R^2$ für eine Hydroxyschutzgruppe stehen, mit einem Alkalisalz einer $C_1$-$C_7$-Carbonsäure umsetzt, das so erhaltene 15α-(1-Hydroxy-2-alkanoyloxyalkyl)-androstan-Derivat in an sich bekannter Weise verestert, durch Bromierung und Bromwasserstoffabspaltung Doppelbindungen in 1,2- und 4,5-Stellung einführt, die 17-O-Schutzgruppe abspaltet und anschließend zum 17-Keton oxidiert.

Als Hydroxyschutzgruppen kommen insbesondere Alkanoylgruppen mit 2 bis 7 Kohlenstoffatoem infrage. Sie können zum Beispiel mit einer anorganischen Base in alkoholischer Lösung verseift werden.

Die Öffnung des Epoxides zum 1-Hydroxy-2-alkanoyloxyalkyl-Derivat wird durch Umsetzung mit dem Alkalisalz, zum Beispiel dem Natrium- oder Lithiumsalz, der gewünschten $C_1$-$C_7$-Carbonsäure nach literaturbekannten Methoden (zum Beispiel C. Djerassi, Steroid Reactions, San Francisco 1963, 615; Weygand/Hilgetag, Organisch-chemische Experimentierkunst, Leipzig 1970, 667) vorgenommen.

Auch die anderen Reaktionen wie Veresterung der Hydroxygruppen, Einführung der Doppelbindungen in die 1,2- und 4,5-Stellung des Steroidgerüstes und Oxidation der 17-Hydroxygruppe werden nach dem Fachmann bekannten gängigen Literaturverfahren, beispielsweise nach den in der deutschen Offenlegungsschrift 35 39 244 beschriebenen Verfahren, durchgeführt.

Die zu verabreichende Menge der Verbindung schwankt innerhalb eines weiten Bereichs und kann jede wirksame Menge abdecken. In Abhängigkeit des zu behandelnden Zustands und der Art der Verabreichung kann die Menge der verabreichten Verbindung 0,001 - 100 mg/kg Körpergewicht, vorzugsweise 0,01-20 mg/kg Körpergewicht, je Tag betragen.

Zur oralen Verabreichung kommen Kapseln, Pillen, Tabletten, Dragees usw. infrage. Die Dosierungseinheiten können neben dem Wirkstoff einen pharmazeutisch verträglichen Träger, wie zum Beispiel Stärke, Zucker, Sorbit, Gelatine, Gleitmittel, Kieselsäure, Talkum usw., enthalten. Die einzelnen Dosierungseinheiten für die orale Applikation können beispielsweise 10 bis 100 mg des Wirkstoffs (Aromataseinhibitors) enthalten.

Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers. eines oberflächenaktiven Mittels, eines Suspendier- oder Emulgiermittels verwendet. Beispiele für verwendete Öle sind zum Beispiel Olivenöl, Erdnußöl, Baumwollsamenöl, Sojabonhenöl, Rizinusöl und Sesamöl.

Die Verbindungen lassen sich auch in Form einer Depotinjektion oder eines Implantatpräparats anwenden, die so formuliert sein können, daß eine verzögerte Wirkstoff-Freigabe ermöglicht wird.

Implantate können als inerte Materialien zum Beispiel biologisch abbaubare Polymere enthalten oder synthetische Silikone wie zum Beispiel Silikonkautschuk. Die Wirkstoffe können außerdem zur perkutanen Applikation zum Beispiel in ein Pflaster eingearbeitet werden.

Die Erfindung betrifft somit auch pharmazeutische Präparate und die Verwendung der neuen Verbindungen der allgemeinen Formel I zur Herstellung dieser Präparate zur Behandlung von östrogenen-bedingten Krankheiten.

**Beispiel 1**

1,7 g 1-Methyl-15α-3[(1R,2S)-dihydroxypropyl]-androsta-1,4-dien-3,17-dion (deutsche Offenlegungsschrift 35 39 244.) werden in 30 ml Pyridin mit 15 ml Acetanhydrid 30 Minuten auf dem Dampfbad erhitzt, in Eiswasser gefällt, abgesaugt, mit Wasser gewaschen und getrocknet. Nach Umkristallisation aus Ether erhält man 1,6 g 1-Methyl-15α-[(1R,2S)-diacetoxypropyl]-androsta-1,4-dien-3,17-dion.
Schmelzpunkt: 184-185°C.

**Beispiel 2**

Analog der in Beispiel 1 angegebenen Vorschrift wird 1-methyl-15α-[(1R,2S)-diacetoxybutyl]-androsta-1,4-dien-3,17-dion vom Schmelzpunkt 223-224°C erhalten.

**Beispiel 3**

Analog der in Beispiel 1 angegebenen Vorschrift wird 1-Methyl-15α-[(1R,2S)-dibutyryloxypropyl]-androsta-1,4-dien-3,17-dion vom Schmelzpunkt 95-97°C erhalten.

**Beispiel 4**

Zu 26 g n-Hexyltriphenylphosphoniumbromid in 200 ml Dimethylsulfoxid gibt man unter Inertgas 7 g Kalium-tert.-butylat, rührt 15 Minuten und tropft dazu die Lösung von 8,38 g 17β-Hydroxy-1α-methyl-3α-tetrahydropyranyloxy-5α-androstan-15α-carbaldehyd (deutsche Offenlegungsschrift 35 39 244) in 60 ml Dimethylsulfoxid zu und rührt 10 Minuten bei Raumtemperatur. Dann wird zwischen Kochsalzlösung und Methylenchlorid verteilt, die organische Phase eingedampft und der Rückstand an Kieselgel mit Methylenchlorid/Essigester chromatographiert. Man isoliert 8,1 g 15α-Heptenyl-17β-hydroxy-1α-methyl-3α-tetrahydropyranyloxy-5α-androstan. Die Umsetzung zum gewünschten Endprodukt 15α-(1,2-Diacetoxyheptyl)-1-methyl-androsta-1,4-dien-3,17-dion erfolgt nach der in der deutschen Offenlegungschrift 35 39 244 beschriebenen Methode.
Dies Ausbeute beträgt 450 mg.

**Beispiel 5**

Analog der in Beispiel 4 angegebenen Vorschrift wird ausgehend von n-Nonyltriphenylphosphoniumbromid und 17β-Hydroxy-1α-methyl-3α-tetrahydropyranyloxy-5α-androstan-15α-carbaldehyd das 15α-(1,2-Diacetoxydecyl)-1-methyl-androsta-1,4-dien-3,17-dion hergestellt.

**Beispiel 6**

Analog der in Beispiel 4 angegebenen Vorschrift wird ausgehend von 2-Methylpropyl-triphenylphosphoniumbromid und 17β-Hydroxy-1α-methyl-3α-tetrahydropyranyloxy-5α-androstan-15α-carbaldehyd das 15α-(1,2-Diformyloxy-3-methylbutyl)-1-methyl-androstan-1,4-dien-3,17-dion hergestellt. Die als letzte Stufe durchgeführte Formylierung wird dabei mit dem gemischten Anhydrid aus Essigsäure und Ameisensäure durchgeführt.

**Beispiel 7**

740 mg 17β-Acetoxy-15α-(1,2-epoxypropyl)-1α-methyl-5α-androstan-3-on (deutsche Offenlegungsschrift 35 39 244, Beispiel 15c) werden in 20 ml Eisessig gelöst, mit 2 g Natriumacetat versetzt und eine Stunde auf 80°C erhitzt. Man fällt anschließend mit Wasser, saugt die Kristalle ab und trocknet sie. Das Rohprodukt wird an Kieselgel mit Aceton/Hexan chromatographiert. Man erhält 630 mg 17β-Acetoxy-15α-(2-acetoxy-1-hydroxy-propyl)-1α-methyl-5α-androstan-3-on, die anschließend in 5 ml Pyridin gelöst und mit 1 ml Propionylchlorid bei Raumtemperatur eine Stunde gerührt werden. Nach Aufarbeitung und Chromatographie, wie vorstehend beschrieben, erhält man 325 mg 17β-Acetoxy-15α-(2-acetoxy-1-pro-pionyloxy-propyl)-1α-methyl-5α-androstan-3-on. Die Umsetzung Endprodukt 15α-(2-Acetoxy-1-propionyloxy-propyl)-1-methyl-androsta-1,4-dien-3,17-dion erfolgt nach der in der deutschen Offenlegungsschrift 35 32 944 beschriebenen Methode.
Die Ausbeute beträgt 90 mg.

## Patentansprüche

1.) 1-Methyl-15α-alkyl-androsta-1,4-dien-3,17-dione der allgemeinen Formel I

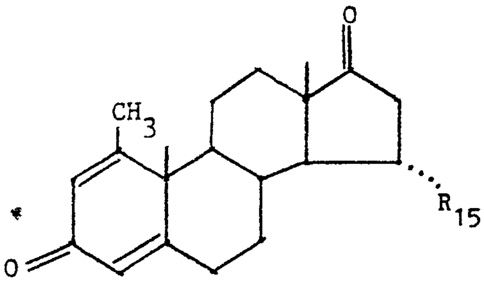

(I),

worin
$R_{15}$ eine geradkettige oder verzweigte Alkylgruppe mit 2 bis 10 Kohlenstoffatomen darstellt, die in 1-und 2-Stellung durch zwei $C_1$-$C_7$-Alkanoyloxygruppen, die gleich oder verschieden sein können, substituiert ist.

2.) 1-Methyl-15α-(1R,2S-diacetoxypropyl)-androsta-1,4-dien-3,17-dion.
1-Methyl-15α-(1R,2S-diacetoxybutyl)-androsta-1,4-dien-3,17-dion.
1-Methyl-15α-(1R,2S-dibutyryloxypropyl)-androsta-1,4-dien-3,17-dion.
15α-(1,2-Diacetoxyheptyl)-1-methyl-androsta-1,4-dien-3,17-dion.
15α-(1,2-Diacetoxydecyl)-1-methyl-androsta-1,4-dien-3,17-dion.
15α-(1,2-Diformyloxy-3-methylbutyl)-1-methyl-androstan-1,4-dien-3,17-dion.
15α-(2-Acetoxy-1-propionyloxy-propyl)-1-methyl-androsta-1,4-dien-3,17-dion.

3.) Pharmazeutische Präparate, gekennzeichnet durch den Gehalt an einer Verbindung gemäß Anspruch 1 und 2.

4.) Verwendung der Verbindungen gemäß Anspruch 1 und 2 zur Herstellung von Präparaten zur Behandlung von östrogen-bedingten Krankheiten.

5.) Verfahren zur Herstellung von 1-Methyl-15α-alkyl-androsta-1,4-dien-3,17-dionen der allgemeinen Formel I

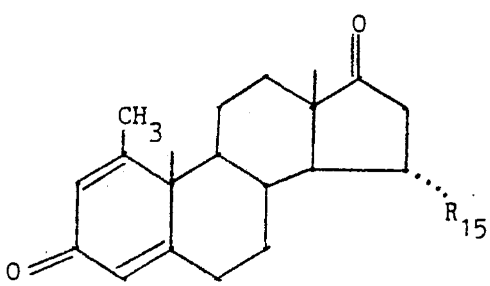

(I),

worin
$R_{15}$ eine geradkettige oder verzweigte Alkylgruppe mit 2 bis 10 Kohlenstoffatomen darstellt, die in 1-und 2-Stellung durch zwei $C_1$-$C_7$-Alkanoyloxygruppen, die gleich oder verschieden sein können, substituiert ist, dadurch gekennzeichnet, daß man

a) 1-Methyl-15α-alkylandrosta-1,4-dien-3,17-dione der allgemeinen Formel II

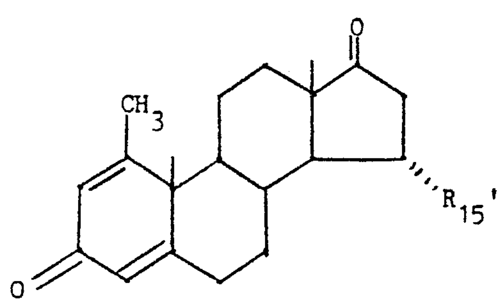

(II),

worin
$R_{15}'$ eine Alkylgruppe mit 2 bis 10 Kohlenstoffatomen darstellt, die in 1- und 2-Stellung durch zwei Hydroxygruppen substituiert ist, in an sich bekannter Weise verestert oder

b) 15α-(1,2-Epoxyalkyl)-1α-methyl-5α-androstan-3-one der allgemeinen Formel III

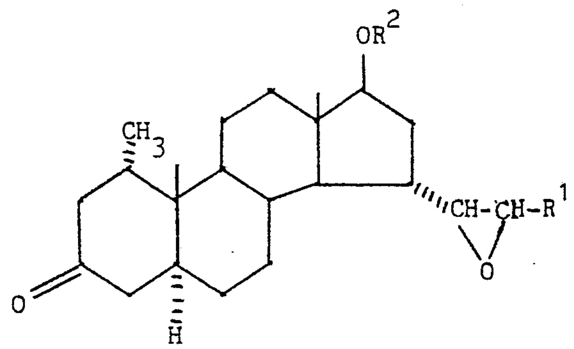 (III) ,

worin
$R^1$ für ein Waserstoffatom oder eine geradkettige oder verzweigte Alkylgruppe mit bis zu 8 Kohlenstoffatomen und $R^2$ für eine Hydroxyschutzgruppe stehen, mit einem Alkalisalz einer $C_1$-$C_7$-Carbonsäure umsetzt, das so erhaltene 15α-(1-Hydroxy-2-alkanoyloxyalkyl)-androstan-Derivat in an sich bekannter Weise verestert, durch Bromierung und Bromwasserstoffabspaltung Doppelbindungen in 1,2-und 4,5-Stellung einführt, die 17-O-Schutzgruppe abspaltet und anschließend zum 17-Keton oxidiert.

Europäisches Patentamt

# EUROPÄISCHER RECHERCHENBERICHT

Nummer der Anmeldung

EP 88 73 0023

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | EP-A-0 129 500 (SCHERING AG)<br>* Ansprüche 1,9 *<br>--- | 1,3,4 | C 07 J 1/00<br>A 61 K 31/565 |
| A | DE-A-3 338 212 (SCHERING AG)<br>* Ansprüche 1,4 *<br>--- | 1,3,4 | |
| P,X<br>D | EP-A-0 225 272 (SCHERING AG)<br>* Ansprüche 1,6,8-10 *<br>----- | 1,3-5 | |
| | | | RECHERCHIERTE SACHGEBIETE (Int. Cl.4) |
| | | | C 07 J 1/00 |

Der vorliegende Recherchenbericht wurde für alle Patentansprüche erstellt

| Recherchenort | Abschlußdatum der Recherche | Prüfer |
|---|---|---|
| DEN HAAG | 25-04-1988 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument